# EUROPEAN PATENT SPECIFICATION

(11) **EP 1 640 402 B1**
(45) Date of publication and mention of the grant of the patent: **11.02.2009**
(21) Application number: 04746258.5
(22) Date of filing: 16.06.2004
(51) Int. Cl.: C08G 73/10, C07D 241/42, G02F 1/1337, H01L 29/786, H05B 33/14, H05B 33/22

(54) **DIAMINE HAVING QUINOXALINE UNIT, POLYIMIDE PRECURSOR, POLYIMIDE AND USE THEREOF**
DIAMIN MIT CHINOXALIN-EINHEIT, POLYIMIDVORLÄUFER, POLYIMID UND VERWENDUNG DAVON
DIAMINE A UNITE QUINOXALINE, PRECURSEUR POLYIMIDE, POLYIMIDE ET UTILISATION

(30) Priority: 17.06.2003 JP 2003172126
(43) Date of publication of application: 29.03.2006
(73) Proprietor: Nissan Chemical Industries, Ltd., Chiyoda-ku, Tokyo 101-0054 (JP)
(72) Inventor: NAGASAKI, Yukio, Moriya-shi, Ibaraki 3020128 (JP); FURUSHO, Hitoshi; Chemical Research Laboratories, Funabashi-shi, Chiba 274-8507 (JP); MIYAMOTO, Hisae; c/o Chemical Research Laboratorie, cho, Funabashi-shi Chiba 2748507 (JP); CHIKAMA, Katsumi; c/o Chemical Research Laborator, Funabashi-shi Chiba 2748507 (JP)
(74) Representative: Stoner, Gerard Patrick
(86) International application number: PCT/JP2004/008789
(87) International publication number: WO 2004/111108

(56) References cited:
- EP-A1- 0 420 417
- WO-A1-02/103825
- JP-A- 2 269 124
- JP-A- 6 316 635
- JP-A- 2001 035 494

## Description

### TECHNICAL FIELD

The present invention relates to a diaminobenzene compound, a polyimide precursor or a polyimide synthesized from the diaminobenzene compound, and usage of the polyimide in the form of thin film. More particularly, the present invention relates to a diamine compound with quinoxaline units and also to a polyimide precursor or a polyimide synthesized from the diamine compound. The diamine compound is useful as a monomer for industrial production of a charge carrier transporting polymer.

### BACKGROUND ART

Polyimide is a linear polymer obtained by reaction between a tetracarboxylic acid dianhydride and a diamine. It is characterized by high tensile strength, high toughness, good electrical insulation, good chemical resistance, and good heat resistance.

For this reason, polyimide is used in the form of heat-resistant film, coating film, adhesive, molding resin, laminate resin, and fiber. Because of its outstanding characteristics, polyimide has found use in a variety of applications including automotive parts, special machine parts, electrical and electronic parts, and spacecraft materials. It has also found use in the field of semiconductor elements and liquid crystal display elements. Examples of its use include insulating film (as disclosed in Japanese Patent Laid-Open No. Hei 5-21705), buffer film (as disclosed in Japanese Patent Laid-Open No. Hei 11-347478), protective film, and alignment layer for liquid crystal display elements.

Conventional polyimide, however, has a disadvantage arising from its high insulating power that leads to static build-up. For example, a polyimide film accumulates electric charges therein upon voltage application. Static charge in polyimide poses many problems with element characteristics and element manufacturing process. Thus, there has been a demand for a polyimide resin which has a low electrical resistance for less charge build-up while retaining its many characteristic properties.

The lowering of polyimide's electrical resistance has been attempted in several ways, which include incorporation with metal powder, conductive metal oxide, or carbon black (as disclosed in Japanese Patent Laid-Open No. 2002-292656) and incorporation with an ionic surface active agent (as disclosed in Japanese Patent Laid-Open No. Hei 7-330650). Polyimide thin film produced by these methods is uneven in thickness, poor in clarity, or heavy with ionic impurities, so that it is not suitable for electronic devices.

Among known conductive polymers (or low-resistance polymeric materials) are polyaniline, polypyrrole, and polythiophene. They are produced respectively from aniline, pyrrole, or thiophen (including their derivatives) as monomer by oxidative polymerization with the help of an oxidizing agent or by electrochemical polymerization.

It is generally known that the thus obtained conductive polymers exhibit high conductivity upon doping with a Lewis acid. The doped conductive polymer finds use as an antistatic agent and an electro-magnetic wave shielding material. (See "Handbook of Conducting Polymers", pp. 13 to 15 and 518 to 529, by Terje A. Skotheim and Ronald L. Elsenbaumer, 2nd ed., 1998, published by Marcel Dekker, Inc.)

EP0420417 A1 discloses a polyimide-poly(phenylquinoxaline) block copolymer prepared by reacting a diamine functionalized poly(phenylquinoxaline) with a tetracarboxylic derivative. The polymer obtained has the requisite properties to perform as dielectric layers in thin film multilayer structures.

Unfortunately, the conductive polymer prepared by polymerization as mentioned above is usually poor in solubility in solvents. Moreover, its solution or dispersion in an organic solvent merely gives a thin film which is poor in mechanical strength. Thus it is difficult to make it into a tough thin film.

Although some conductive polymers are soluble in organic solvents, their solutions (in varnish form) are highly unstable and subject to gelation in most cases.

As mentioned above, conventional polyimide has many shortcomings such as, high insulating performance, easy static charging, and static build-up due to voltage application. On the other hand, conductive polymers free of these shortcomings are still unsatisfactory in solution stability and thin film properties. So, there has been a demand for a new conductive polymer free of these shortcomings.

### DISCLOSURE OF INVENTION

The present invention was completed in view of the foregoing. It is an object of the present invention to provide a diaminobenzene compound which gives a polyimide film (or thin film) which has high heat resistance and low electrical resistance and is capable of charge carrier transportation. It is another object of the present invention to provide a polyimide precursor (or polyamic acid) and a polyimide, which are obtained from the diaminobenzene compound. It is further another object of the present invention to provide the usage of the polyimide thin film.

In order to address the above-mentioned problems, the present inventors carried out extensive studies on how to improve the charge carrier transport of polyimide thin film, how to improve the strength of polyimide coating film, and how to improve the stability of polyimide varnish. As the result, it was found that a polyimide and a precursor thereof which are derived from a diaminobenzene compound represented by formula (1), which has quinoxaline units (or which has quinoxaline skeletons in the main chain), exhibit stable electrical and mechanical properties. This finding led to the present invention.

The present invention covers the following aspects [1] to [13].
[1] A diaminobenzene compound represented by formula (1) below. 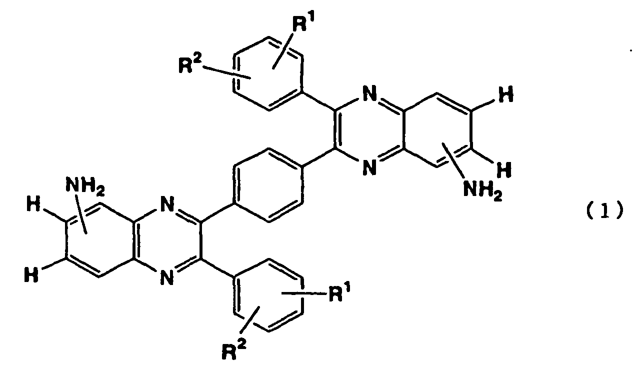 (where R¹ and R² each independently denotes a hydrogen atom, alkyl group, or alkoxyl group.)
[2] The diaminobenzene compound as defined in [1], wherein R¹ and R² each independently denotes a C₁₋₂₀ alkyl group, C₁₋₂₀ alkoxyl group, or C₁₋₂₀ fluoroalkyl group.
[3] A polyimide precursor which includes repeating units represented by formula (2) below. 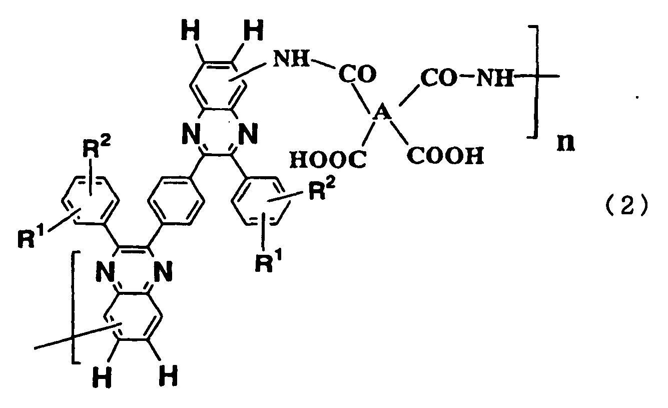 (where R¹ and R² each independently denotes a hydrogen atom, alkyl group, or alkoxyl group; "A" denotes a residue of tetracarboxylic acid; and n denotes an integer of 1 to 5000.)
[4] A polyimide which includes repeating units represented by formula (3) below. 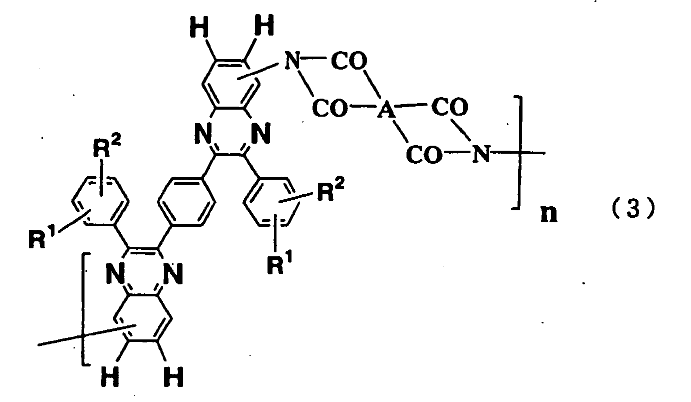 (where R¹ and R² each independently denotes a hydrogen atom, alkyl group, or alkoxyl group; "A" denotes a residue of tetracarboxylic acid; and n denotes an integer of 1 to 5000.)
[5] A polyimide precursor which is obtained by reaction between a diamine component containing at least 1 mol% of the diaminobenzene compound defined in [1] or [2] and a tetracarboxylic acid or a derivative thereof.
[6] The polyimide precursor as defined in [5], wherein the tetracarboxylic acid or the derivative thereof is an aromatic tetracarboxylic acid or a derivative thereof.
[7] The polyimide precursor as defined in [6], wherein the aromatic tetracarboxylic acid is a tetracarboxylic acid having phenyl groups or substituted phenyl groups.
[8] A polyimide which is obtained by ring-closing reaction from any of polyimide precursors as defined in [5] to [7].
[9] A charge carrier transporting film which is formed from the polyimide as defined in [4] or [7].
[10] An organic transistor device which is the charge carrier transporting film as defined in [9].
[11] An organic light-emitting diode which has at least one layer of the charge carrier transporting film as defined in [9].
[12] A fluorescent filter which is the charge carrier transporting film as defined in [9].
[13] A liquid crystal alignment film which is the charge carrier transporting film as defined in [9].

The diaminobenzene compound according to the present invention is easy to synthesize, and it can be made into a polyimide which is excellent in heat resistance, coating film strength, and thin film properties and is capable of charge carrier transportation. The resulting polyimide gives a polyimide film which has a lower electrical resistance than conventional one and is capable of transporting charge carriers. Therefore, the polyimide film will find use as organic transistor device, organic light-emitting diode, fluorescent filter, liquid crystal alignment layer, and other electronic device coatings and electronic materials.

### BRIEF DESCRIPTION OF DRAWINGS

Fig. 1 is a diagram illustrating the dependence on frequency of the integer part (ε) of the polyimide thin film in Example 9;
Fig. 2 is a diagram illustrating the behavior of carriers in the polyimide thin film in Example 9;
Fig. 3 is a diagram illustrating the current-voltage characteristics of the polyimide thin film in Examples 15 to 17; and
Fig. 4 is a diagram illustrating the voltage-luminance characteristics of the organic light-emitting diode with the polyimide thin film in Examples 18 to 20.

### BEST MODE FOR CARRYING OUT THE INVENTION

The invention will be described below in more detail.

According to the present invention, the diaminobenzene compound having quinoxaline units is represented by formula (1). It can be readily synthesized and it finds use as a raw material for polyimide and polyamide.

The diaminobenzene compound represented by formula (1) is composed of a diamine moiety and a quinoxaline moiety. It can be synthesized by any method which is not specifically restricted. A typical example of synthesis method is given below.

First, a nitro compound represented by formula (4) 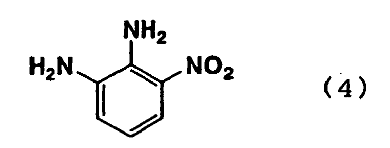 is synthesized, and then it is reacted with a benzyl compound represented by formula (5) in methanol in the presence of acetic acid, 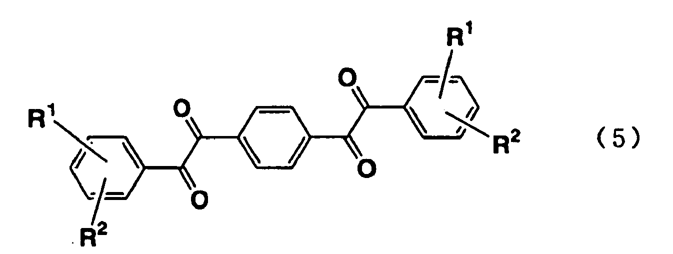 to give a dinitro compound represented by formula (6).

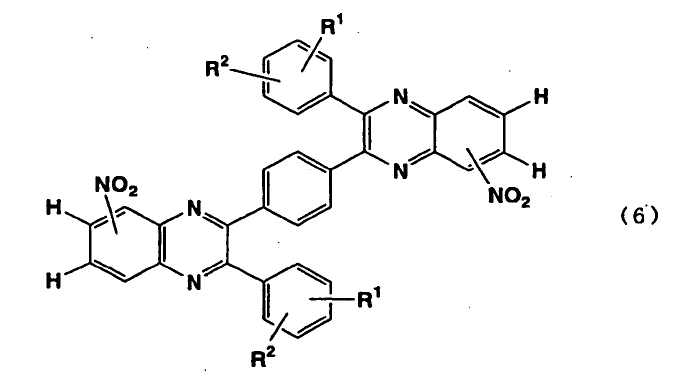

Finally, the reaction product has its nitro groups converted into amino groups by reduction with hydrogen in the presence of palladium carbon catalyst.

The substituent groups R¹ and R² are usually hydrogen, but they may be alkyl, alkoxyl, or fluoroalkyl groups for increased solvent solubility. These groups usually have a carbon number of 1 to 4 but possibly have a carbon number up to 20. Incidentally, in formulas (1) to (6), the substituent groups with identical symbols may be the same or different.

After synthesis as mentioned above, the resulting diaminobenzene compound of the present invention, which is represented by formula (1), undergoes polycondensation with a tetracarboxylic acid or a derivative thereof, such as tetracarboxylic acid dihalide and tetracarboxylic acid dianhydride. Thus there is obtained a polyimide precursor represented by formula (2), which has in the main chain a quinoxaline derivative capable of carrier transportation. 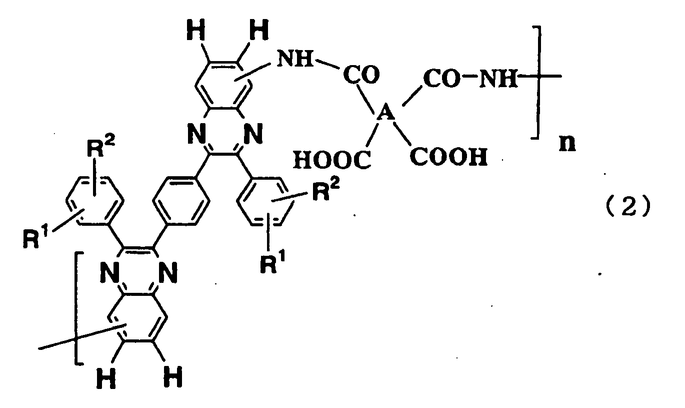 (where "A" denotes a residue of tetracarboxylic acid; and n denotes an integer of 1 to 5000.)

Then, this polyimide precursor undergoes dehydration ring-closing reaction. Thus there is obtained a polyimide having repeating units represented by formula (3). 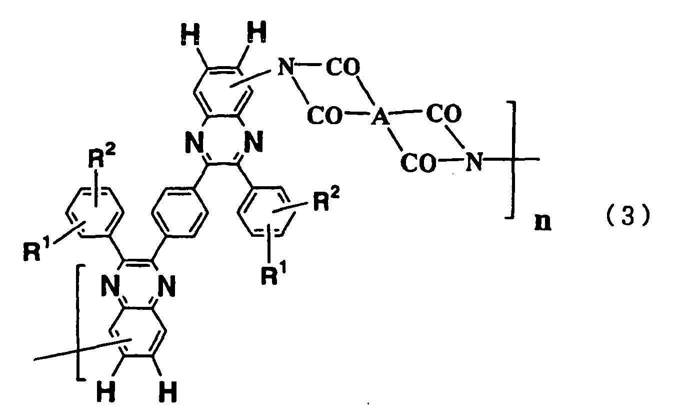 (where "A" and n are defined as above.)

The following are typical examples of the tetracarboxylic acid and derivatives thereof. Aromatic tetracarboxylic acid, dianhydride thereof, and dicarboxylic acid diacyl halide thereof, such as pyromellitic acid, 3,3,3',4,4'-benzophenonetetracarboxylic acid, 3,3',4,4'-biphenyltetracarboxylic acid, 2,3,3',4-biphenyltetracarboxylic acid, bis(3,4-dicarboxyphenyl)ether, bis(3,4-dicarboxyphenyl)sulfone, bis(3,4-dicarboxyphenyl)methane, 2,2-bis(3,4-dicarboxyphenyl)propane, 1,1,1,3,3,3-hexafluoro-2,2-bis(3,4-dicarboxyphenyl)propane, bis(3,4-dicarboxyphenyl)diphenylsilane, 2,3,6,7-napththalenetetracarboxylic acid, 1,2,5,6-naphthalenetetracarboxylic acid, 1,4,5,8-naphthalenetetracarboxylic acid, 2,3,4,5-pyridinetetracaboxylic acid, 2,6-bis(3,4-dicarboxyphenyl)pyridine. Alicyclic tetracarboxylic acid, dianhydride thereof, and dicarboxylic acid diacyl halide thereof, such as 1,2,3,4-cyclobutanetetracarboxlic acid, 1,2,3,4-cyclopentanetetracarboxylic acid, 2,3,5-tricarboxycyclopentylacetic acid, and 3,4-dicarboxy-1,2,3,4-tetrahydro-1-naphthalenesuccinic acid. Aliphatic tetracarboxylic acid, dianhydride thereof, and dicarboxylic acid diacyl halide thereof, such as 1,2,3,4-butanetetracarboxylic acid.

Of the tetracarboxylic acids and derivatives thereof mentioned above, aromatic tetracarboxylic acids and derivatives thereof are preferable, and aromatic tetracarboxylic acids and derivatives thereof having phenyl groups or substituted phenyl groups are more preferable. In the latter case, the substituent group in the substituted phenyl group may be a C₁₋₁₀ alkyl group or alkoxyl group, preferably a C₁₋₅ alkyl group or alkoxyl group. Two or more species of tetracarboxylic acid and derivative thereof may be used in combination.

The polyimide of the present invention may be obtained from any diamine compound which contains as an essential component the diaminobenzene derivative represented by formula (1), which is abbreviated as diamine (1) hereinafter. Copolymerization of diamine (1) and a tetracarboxylic acid derivative or copolymerization of diamine (1), an ordinary diamine other than diamine (1), and a tetracarboxylic acid or derivative thereof yields the desired polyimide which has in the main chain a quinoxaline derivative capable of carrier transportation.

The ordinary diamine that can be used is not specifically restricted so long as it is a primary diamine suitable for polyimide synthesis. Its typical examples are listed below.

Aromatic diamines such as p-phenylenediamine, m-phenylenediamine, 2,5-diaminotoluene, 2,6-diaminotoluene, 4,4'-diaminobiphenyl, 3,3'-dimethyl-4,4'-diaminobiphenyl, diaminodiphenylmethane, diaminodiphenyl ether, 2,2'-diaminodiphenylpropane, bis(3,5-diethyl-4-aminophenyl)methane, diaminodiphenylsulfone, diaminobenzophenone, diaminonaphthalene, 1,4-bis(4-aminophenoxy)benzene, 1,4-bis(4-aminophenyl)benzene, 9,10-bis(4-aminophenyl)anthracene, 1,3-bis(4-aminophenoxy)benzene, 4,4'-bis(4-aminophenoxy)diphenylsulfone, 2,2-bis[4-(4-aminophenoxy)phenyl]propane, 2,2-bis(4-aminophenyl)hexafluoropropane, and 2,2-bis[4-(4-aminophenoxy)phenyl]hexafluoropropane.

Alicyclic diamines such as bis(4-aminocyclohexyl)methane and bis(4-amino-3-methylcyclohexyl)methane.

Aliphatic diamines such as tetramethylenediamine and hexamethylenediamine.

Additional examples include diaminosiloxanes represented by the following formula. 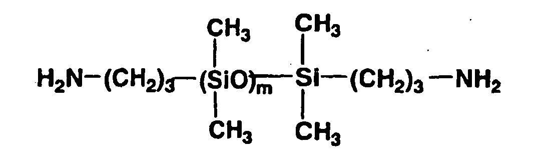 (where m is an integer of 1 to 10.)

These diamines may be used alone or in combination with another.

Diamine (1) may be used in combination with an ordinary diamine for synthesis of the polyimide of the present invention. In this case the amount (in molar ratio) of diamine (1) in the total amount of diamines should be properly controlled so that the resulting polyimide has improved surface characteristics such as water repellency.

The molar ratio of diamine (1) to the total amount of diamines should be no less than 1 mol%, preferably no less than 5 mol%.

According to the present invention, the polyimide precursor is prepared by polymerization reaction between a tetracarboxylic acid (or a derivative thereof) and a combination of diamine (1) and an ordinary diamine. The precursor is made into an imide by ring-closing reaction. The tetracarboxylic acid (or a derivative thereof) for this reaction is usually tetracarboxylic acid dianhydride.

The molar ratio of tetracarboxylic acid dianhydride to diamines (combination of diamine (1) and ordinary diamine) should preferably be from 0.8 to 1.2. As in the case of ordinary polycondensation reaction, the closer to 1 the molar ratio, the larger the molecular weight of the resulting polyimide precursor (or polyimide).

The polyimide precursor (or polyimide) having an excessively small molecular weight will give a coating film poor in strength. Conversely, the polyimide precursor (or polyimide) having an excessively large molecular weight will give a thin film poor in uniformity and workability. Therefore, the polyimide precursor (or polyimide) according to the present invention should have a weight-average molecular weight of 10,000 to 1,000,000 measured by GPC (Gel Permeation Chromatography).

The method for polymerization reaction between tetracarboxylic acid dianhydride and diamine is not specifically restricted. It usually involves dissolving a primary diamine in an organic polar solvent (such as N-methylpyrrolidone, N,N-dimethylacetamide, and N,N-dimethylformamide), adding to the resulting solution a tetracarboxylic acid dianhydride, thereby synthesizing a polyimide precursor, and performing dehydration ring-closing reaction, thereby forming the desired imide.

The reaction between tetracarboxylic acid dianhydride and diamine to form a polyimide precursor should be carried out at temperatures ranging from -20°C to 150°C, preferably from -5°C to 100°C. This polyimide precursor can be converted into a polyimide by dehydration with heating at 100°C to 400°C or by chemical imidization with the help of a catalyst such as triethylamine/acetic anhydride.

The polyimide thin film according to the present invention may be obtained by applying a solution of polyimide precursor to a substrate and then imidizing it by heating on the substrate. The solution of polyimide precursor may be the polymer solution obtained as mentioned above. Alternatively, it may be prepared by placing the polyimide precursor in a large amount of poor solvent, such as water and methanol, for precipitation, recovering precipitates, and dissolving recovered precipitates again in a solvent.

No specific restrictions are imposed on the solvent in which the polyimide precursor is dissolved or the solvent in which recovered precipitates are dissolved again, so long as it dissolves the polyimide precursor. Examples of such solvents include N-methylpyrrolidone, N,N-dimethylacetamide, and N,N-dimethylformamide. They may be used alone or in combination with one another. They may also be combined with any other solvent to give a uniform solvent. Such solvents include ethyl cellosolve, butyl cellosolve, ethyl carbitol, butyl carbitol, ethyl carbitol acetate, and ethylene glycol.

The solution of polyimide precursor may be incorporated with a coupling agent in order to achieve better adhesion between the polyimide thin film and the substrate. Imidization by heating may be accomplished at any temperature ranging from 100°C to 400°C, preferably 150°C to 350°C.

In the case of a solvent-soluble polyimide, it is possible to prepare a polyimide solution by reacting a tetracarboxylic acid dianhydride with a primary diamine and then imidizing the resulting polyimide precursor in the solution. Conversion of the polyimide precursor into the polyimide in the solution may usually be accomplished by heating for dehydration ring-closing reaction. The reaction temperature is 150°C to 350°C, preferably 120°C to 250°C.

It is also possible to convert a polyimide precursor into a polyimide by ring-closing reaction with the help of any catalyst for dehydration ring-closing reaction. The thus obtained polyimide solution may be used as such. Alternatively, it may be placed in a poor solvent (such as methanol and ethanol) for precipitation and the resulting precipitates are dissolved again in an adequate solvent to give the desired solution. No restrictions are imposed on the solvent for redissolution so long as it dissolves the polyimide. Examples of such solvents include 2-pyrrolidone, N-ethylpyrrolidone, N-vinylpyrrolidone, and γ-butyl lactone, in addition to those mentioned above in relation to polyamide precursors. These solvents may be used in combination with other solvents (mentioned above) if their dissolving power is not enough.

The polyimide solution may be incorporated with a coupling agent in order to achieve better adhesion between the polyimide thin film and the substrate. It is possible to form a polyimide thin film on a substrate by applying the polyimide solution onto a substrate and then evaporating the solvent. The temperature for solvent evaporation is usually 80°C to 150°C.

The manner of coating is not critical and includes, for example, a dipping method, a spin coating method, a transferring printing method, a roll coating method, a brushing method, and the like.

### EXAMPLE

The invention will be described in more detail with reference to the following synthesis examples, examples, and comparative examples, which are not intended to restrict the scope thereof.

### Synthesis Example 1

### Synthesis of 1-nitro-2,3-diaminobenzene

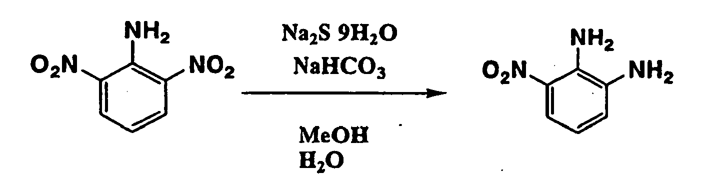

In 225 ml of methanol was dissolved 14 g of commercial 1-amino-2,5-dinitrobenzene. To the solution was added dropwise through a dropping funnel a solution of sodium sulfide (60 g) and sodium hydrogen carbonate (21 g) dissolved in water (240 g), with the reaction temperature kept at 60°C. The reaction solution was stirred at 60°C for one hour. After the completion of reaction, the reaction solution was cooled to room temperature and filtered.
M/z (FD+) 153 (calculated: 153.1396)
¹H-NMR (CDCl₃, δppm) 7.72, 7.70, 7.24, 6.92, 6.62, 6.60, 6.59, 5.92, 3.40
Yield: 7.79 g (yield ratio:66.5%)

### Synthesis Example 2

Synthesis of 1,4-bis[5-nitro-3-phenylquinoxalin-2-yl]benzene

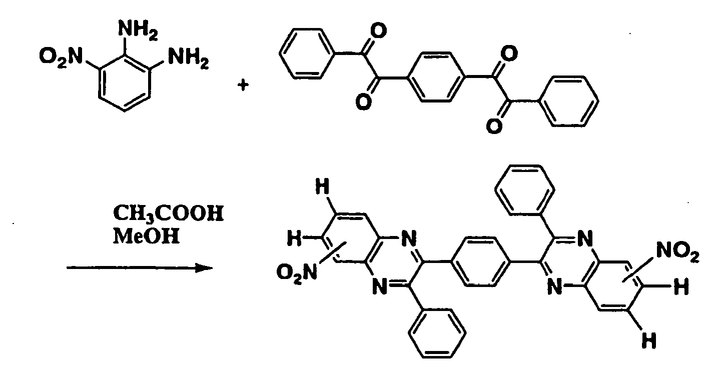

In a 1:1 mixed solvent of acetic acid and methanol were dispersed 1-nitro-2,3-diaminobenzene (3.06 g) and 1,4-bisbenzyl. The resulting dispersion was stirred at 60°C for 3.5 hours under a nitrogen atmosphere. After the completion of reaction, the reaction system was cooled to room temperature. Precipitates were filtered off and washed with methanol. Upon drying, there was obtained the desired product.
M/z (FD+) 575 (calculated: 576.56)
Yield: 6.35 g (yield ratio: 95%)

### Example 1

### Synthesis of 1,4-bis[5-amino-3-phenylquinoxalin-2-yl]benzene

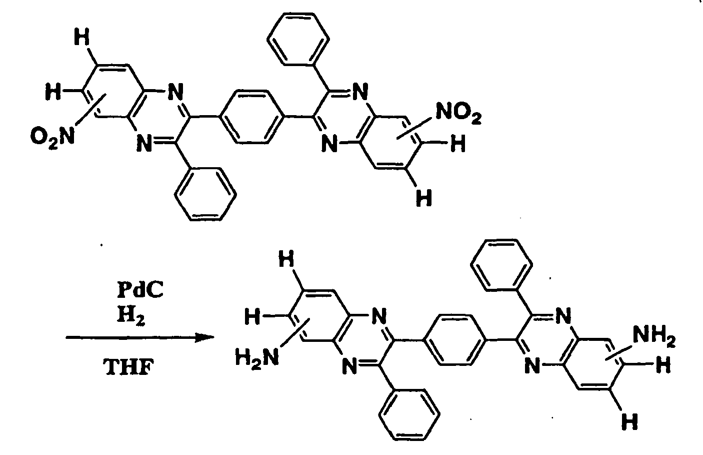

In THF (300 g) was dissolved 1,4-bis[5-nitro-3-phenylquinoxanlin-2-yl]benzene (2.37 g). With the atmosphere replaced with nitrogen, PdC (1.14 g) was added. The atmosphere in the reaction system was replaced with nitrogen. Then, a prescribed amount of hydrogen was added, and stirring was continued at room temperature for 48 hours. After the completion of reaction, precipitates were filtered off and washed with methanol until washings became colorless. After drying, the precipitates were purified by passing through a silica gel column to give the desired product. The eluent was chloroform.
M/z (FD+) 515 (calculated: 516.59)
Yield: 1.05 g (yield ratio: 49.5%)

### Examples 2 to 4

### Synthesis of polyimide precursor

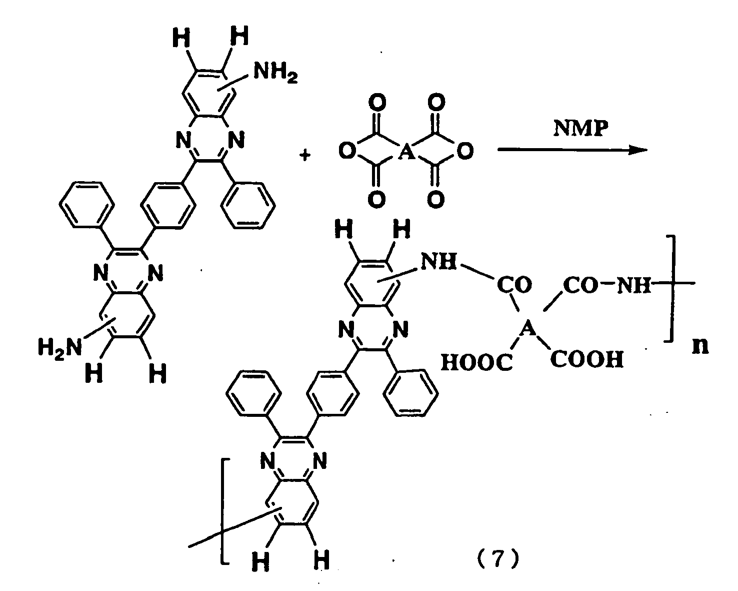

An acid dianhydride represented by one of the following formulas 1 to 3 was reacted with 1,4-bis[5-amino-3-phenylquioxalin-2-yl]benzene in NMP with stirring under the conditions shown in Table 1. This reaction gave the polyimide precursor represented by formula (7), where "A" denotes a residue of tetracarboxylic acid. Table 1 also shows the conditions of polymerization and the molecular weight of the resulting polymer.

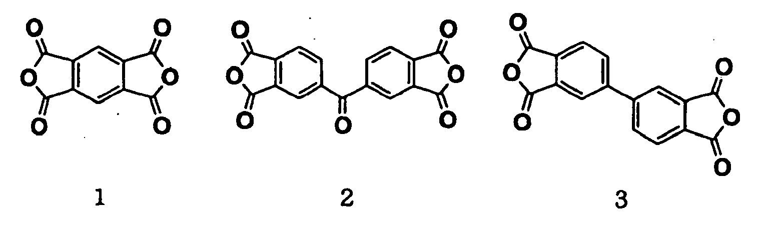

**Table 1**

| Example | Acid dianhydride (mol) | Diamine (mol) | *¹ Solvent | Polymerization conditions Temperature (°C) Time (hr) | Molecular weight GPC (Peak top) GPC (Peak top) Av. mol. weight (Mn) |
|---|---|---|---|---|---|
| 2 | 1 (1.9 mmol) | 1.9 mmol | NMP | 25 144 | 24539 (Mpeak top) 22488 (Mn) |
| 3 | 2 (5.0mmol) | 5.0mmol | NMP | 25 144 | 47950 (Mpeak top) 36493 (Mn) |
| 4 | 3 (5.0 mmol) | 5.0mmol | NMP | 25 144 | 25941 (Mpeak top) 22495 (Mn) |

| | | | | | |
|---|---|---|---|---|---|
| *1: Concentration of solids 20% by weight (After reaction, the concentration of solids was adjusted to 5% by weight by adding NMP.) | | | | | |

### Synthesis Example 3

### Synthesis of 1,3-bis[(4-tert-butylphenyl)glyoxaloyl]benzene

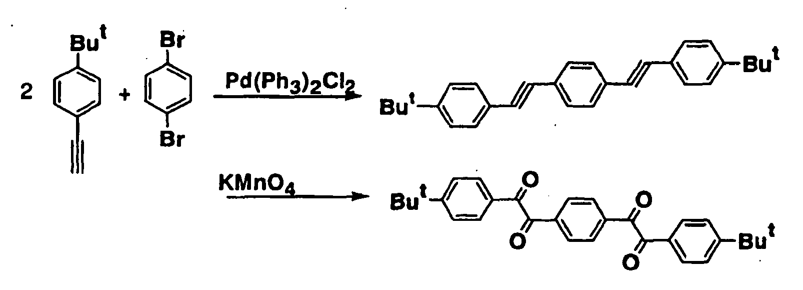

2.66 ml of 1,4-dibromobenzene was dissolved in triethylamine (70 ml) and pyridine (30 ml). With dissolved oxygen removed, Pd(Ph₃)₂Cl₂ (0.3 g), triphenylsulfone (0.6 g), and copper iodide (CuI) (0.1 g) were added. Dissolved oxygen was removed again. To the solution was added dropwise with stirring a solution of 4-tert-butylphenylacetylene (9 ml) dissolved in pyridine (9 ml). The reaction temperature was kept at 70°C. Reaction was continued for 24 hours at 70°C. An excess amount of dilute hydrochloric acid was added to the reaction product to cause precipitation. Precipitates were filtered off and extracted by chloroform. The crude product separated by filtration was washed with dilute hydrochloric acid and water. The washed crude product was recrystallized from 2-propanol. Thus there was obtained 1,3-bis[4-tert-butylphenylethynyl]benzene in the form of colorless crystals.
M/z (FD+) 389 (calculated: 390.56)

One gram of the colorless crystals was dissolved in 400 ml of acetone. The resulting solution was added to a solution of sodium hydrogen carbonate (0.5 g) and magnesium sulfate (2.5 g) dissolved in 60 ml of water. To the thus obtained solution was added 2.3 g of potassium permanganate, and the reaction solution was stirred for 4 hours. The reaction solution was acidified with dilute sulfuric acid. NaNO₂ was added to suspend the oxidation reaction. Manganese oxide was filtered off, and the desired product was extracted with a 1:1 mixed solvent of hexane and ether. Upon recrystallization from ethanol, there were obtained yellowish crystals (yield ratio: 83%).
M/z (FD+) 453 (calculated 454.56)

### Synthesis Example 4

### Synthesis of 1,4-bis[3-(4-tert-butylphenyl)-5-nitroquinoxalin-2-yl]benzene

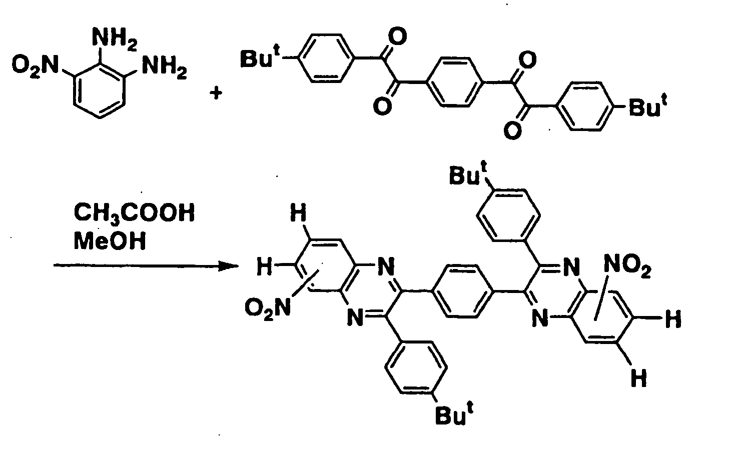

In a 1:1 mixed solvent of acetic acid and methanol were dispersed 3.06 of 1-nitro-2,3-diaminobenzene and 9.10 g of 1,3-bis[(4-tert-butylphenyl)glyoxyaloyl]benzene. The dispersion was stirred under a nitrogen atmosphere at 60°C for 3.5 hours. After the completion of reaction, the reaction system was cooled to room temperature, and precipitates were filtered out and washed with methanol. Upon drying, there was obtained the desired product.
M/z (FD+) 687 (calculated: 688.77)
Yield: 5.35 g (yield ratio: 38.9%)

### Example 5

### Synthesis of 1,4-bis[3-(4-tert-butylphenyl)-5-aminoquinoxalin-2-yl]benzene

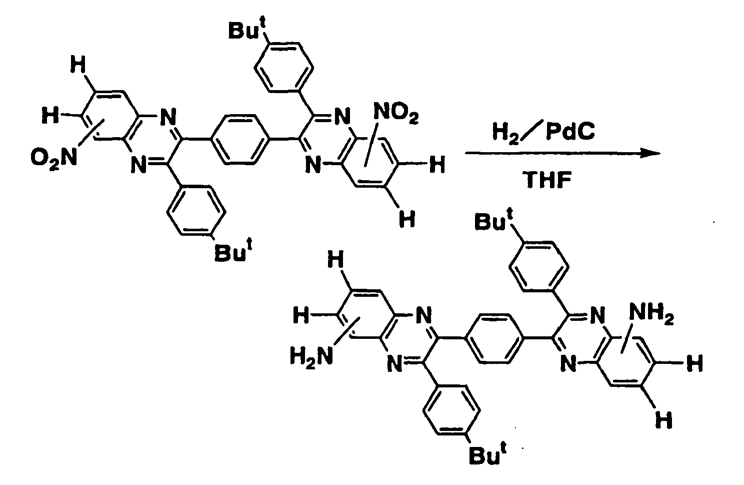

In THF (300 g) was dissolved 3.44 g of 1,4-bis[3-(4-tert-butylphenyl)-5-nitroquinoxalin-2-yl]benzene. With atmosphere in the reaction system replaced with nitrogen, 1.14 g of PdC was added. With the atmosphere in the reaction system replaced with nitrogen, a prescribed amount of hydrogen was added. Stirring was continued at room temperature for 48 hours. After the completion of reaction, precipitates were filtered off and washed with methanol until washings became colorless. After drying, the precipitates were purified by using a silica gel column. Thus there was obtained the desired product in the form of yellowish fine powder. The eluent was chloroform.
M/z (FD+) 627 (calculated: 628.81)
IR: 3350 cm⁻¹ (νNH), 1550 cm⁻¹ (νNO), 1370 cm⁻¹ (νNO), 1320 cm⁻¹ (νCN), 1220 cm⁻¹ (νCO), 820 cm⁻¹ (1,4-di-substituted benzene)

### Examples 6 to 8

### Synthesis of polyimide precursor

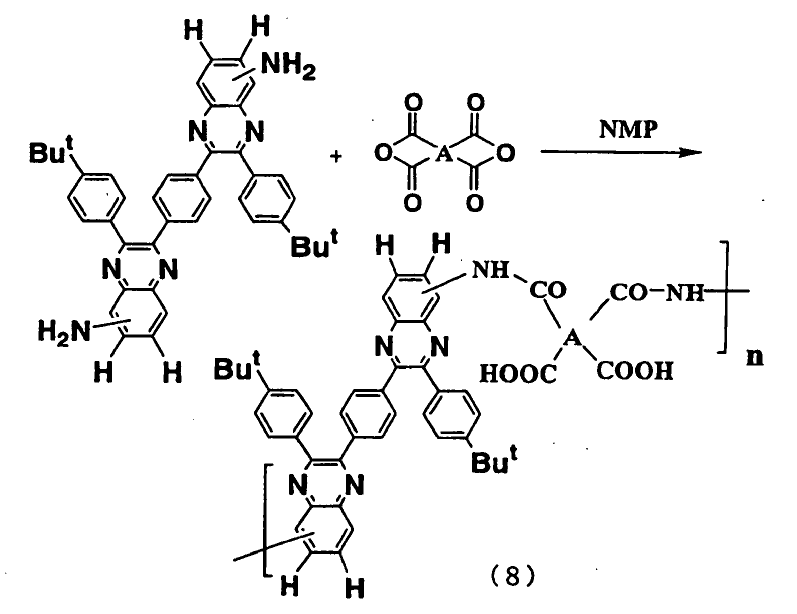

A tetracarboxylic acid dianhydride represented by one of the following formulas 1 to 3 was reacted with 1,4-bis[3-(4-tert-butylphenyl)-5-aminoquinoxalin-2-yl]benzene in NMP with stirring under the conditions shown in Table 2. This reaction gave the polyimide precursor represented by formula (8), where "A" denotes a residue of tetracarboxylic acid. Table 2 also shows the conditions of polymerization and the molecular weight of the polymer.

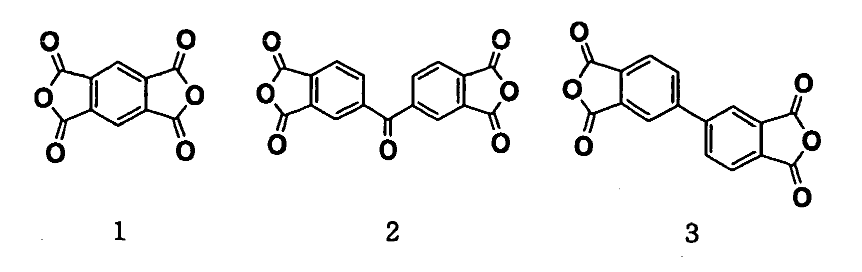

**Table 2**

| Example | Acid dianhydride (mol) | Diamine (mol) | *¹ Solvent | Polymerization conditions Temperature (°C) Time (hr) | Molecular weight GPC (Peak top) Av. mol. weight (Mn) |
|---|---|---|---|---|---|
| 6 | 1 (5.0 mmol) | 5.0 mmol | NMP | 25 144 | 35760 (Mpeak top) 23495 (Mn) |
| 7 | 2 (5.0mmol) | 5.0 mmol | NMP | 25 144 | 57430 (Mpeak top) 42382 (Mn) |
| 8 | 3 5.0 mmol | 5.0 mmol | NMP | 25 144 | 36580 (Mpeak top) 24256 (Mn) |

| | | | | | |
|---|---|---|---|---|---|
| *1: Concentration of solids 20% by weight (After reaction, the concentration of solids was adjusted to 5% by weight by adding NMP.) | | | | | |

### Examples 9 to 14

### Charge transporting capability of polyimide thin film, and evaluation of charge carrier transporting film

Each sample of the polyimide precursors (in varnish form) obtained in Examples 2 to 4 and 6 to 8 was applied to a cleaned ITO-coated glass substrate by spin coating. The coating step was followed by baking at 200°C for one hour. There was obtained a polyimide thin film. This thin film was provided with an aluminum electrode by vapor deposition. The thin film was tested for impedance to confirm that it was correctly formed. The charge transporting capability in the thin film was examined by the TOF method.

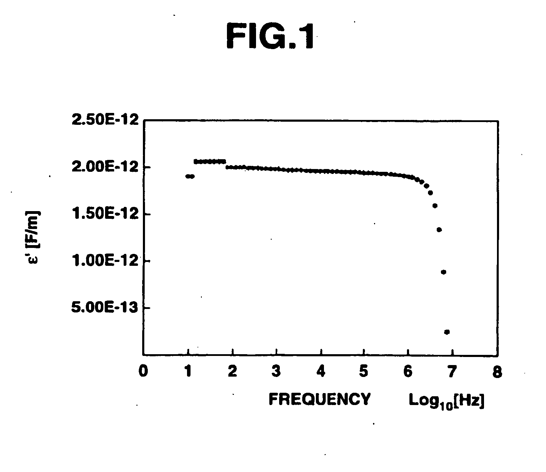

The graph in Fig. 1 shows the frequency dependence of the integer part (ε) of the polyimide thin film (obtained from the polyimide precursor varnish in Example 9). This result suggests that the thin film was correctly formed.

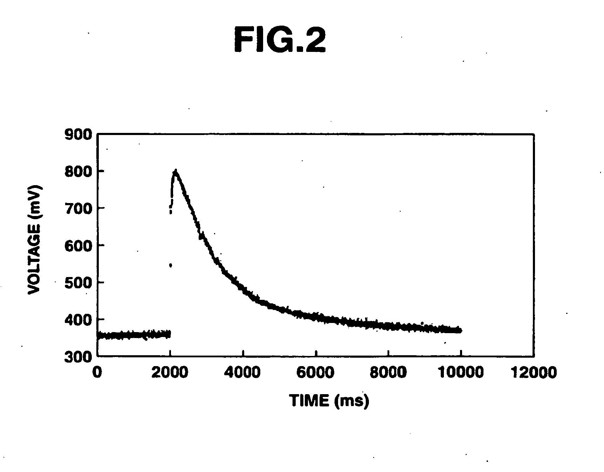

All the samples of the polyimide thin film in Examples 9 to 14 gave the same results as mentioned above. Thus, they were tested by the TOF method for their ability to transport carriers therein. The graph in Fig. 2 typically shows the behavior of carriers in the polyimide thin film.

It is noted from the graph in Fig. 2 that carriers run for about 200 µ second in the thin film. Table 3 below shows the running time of carriers in the sample of the polyimide thin film in Examples 9 to 14.

**Table 3**

| Polyimide | Polyimide precursor | Running time (µ sec) |
|---|---|---|
| Example 9 | Example 2 | 200 |
| Example 10 | Example 3 | 150 |
| Example 11 | Example 4 | 185 |
| Example 12 | Example 6 | 210 |
| Example 13 | Example 7 | 175 |
| Example 14 | Example 8 | 200 |

### Examples 15 to 17

### Evaluation of the rectifying characteristics of polyimide thin film

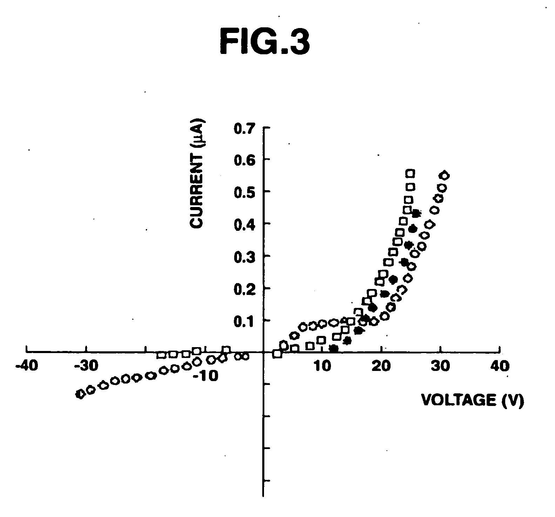

Each sample of the polyimide precursor varnishes obtained in Example 2 to 4 was applied to a cleaned ITO-coated glass substrate by spin coating. The coating step was followed by baking at 200°C for one hour. Thus there was obtained a polyimide thin film. On this thin film was formed an aluminum electrode by vapor deposition. The thin film was tested for impedance to confirm that it was correctly formed. The thin film was further tested for current-voltage characteristics. It was found that the thin film exhibits the rectifying characteristics. The results are shown in Fig. 3.

The element tested has a laminate structure composed of an ITO electrode, a polyimide layer (formed by spin coating), and an aluminum layer (formed by vapor deposition) which are placed one over another.

Incidentally, in Fig. 3, "●" denotes the polyimide thin film of Example 15 (which is obtained from the polyimide precursor varnish of Example 2), "○" denotes the polyimide thin film of Example 16 (which is obtained from the polyimide precursor varnish of Example 3), and "■" denotes the polyimide thin film of Example 17 (which is obtained from the polyimide precursor varnish of Example 4).

### Examples 18 to 20

### Organic light-emitting diode with polyimide thin film

A sample of organic light-emitting diode was prepared in the following manner with the polyimide precursor varnish obtained in Examples 2 to 4.

A thin film of polyaniline (which is an electroconductive polymer) was formed on an ITO electrode. Onto the thin film was applied by spin coating each sample of polyimide precursors of Examples 2 to 4, so that a 50 nm thick film was formed.

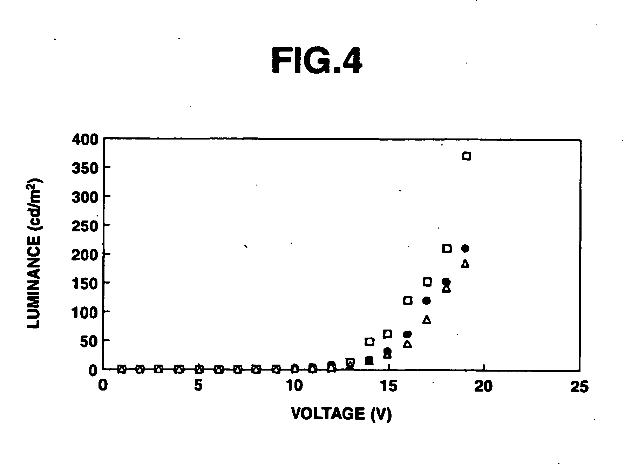

The spin-coated film was baked at 200°C for one hour for ring-closing imidization. On the spin-coated film was formed by vapor deposition a 50 nm thick film of aluminum quinone (Alq₃) (which is an electron transporting material). Finally, a cathode of magnesium-silver was formed by vapor deposition. The cathode was coated with a 500 nm thick aluminum film for protection against oxidation. The resulting element exhibited the voltage-luminance characteristics as shown in Fig. 4.

Incidentally, in Fig. 4, "●" denotes the polyimide thin film of Example 18 (which is obtained from the polyimide precursor varnish of Example 2), "Δ" denotes the polyimide thin film of Example 19 (which is obtained from the polyimide precursor varnish of Example 3), and "■" denotes the polyimide thin film of Example 20 (which is obtained from the polyimide precursor varnish of Example 4).

### Examples 21 to 23

### Fluorescence characteristics of polyimide thin film

Each sample of the polyimide precursor varnish (solution in polyamic acid) of Examples 2 to 4 was tested for fluorescence. Also, each sample of the polyimide precursor varnish in Examples 2 to 4 was made into a thin film by spin coating and ensuing baking at 200°C for one hour, and the resulting thin film was tested for fluorescence. The results are shown in Table 4. The concentration of the polyimide precursor varnish (solution in polyamic acid) is as follows.
Example 2: 3.16 × 10⁻⁶ mol/l
Example 3: 3.32 × 10⁻⁶ mol/l
Example 4: 3.31 × 10⁻⁶ mol/l

**Table 4**

| Example | Fluorescence characteristics of solution | | | Example for polyimide thin film | Fluorescence characteristics of thin film | | |
|---|---|---|---|---|---|---|---|
| | Excitation wavelength (nm) | Fluorescence wavelength (nm) | Intensity (nm) | | Excitation wavelength (nm) | Fluorescence wavelength (nm) | Intensity (nm) |
| 2 | 308 | 489 | 940 | 21 | 315 | 315-600 | 100-250 |
| 3 | 306 | 486 | 498 | 22 | 290 | 300-570 | 200-600 |
| 4 | 308 | 487 | 920 | 23 | 290 | 300-570 | 1000-8000 |

It is noted from Table 4 that all the samples emit intense fluorescence. It is also noted that the polyimide thin film emits white light.

## Claims

1. A diaminobenzene compound represented by formula (1) below. 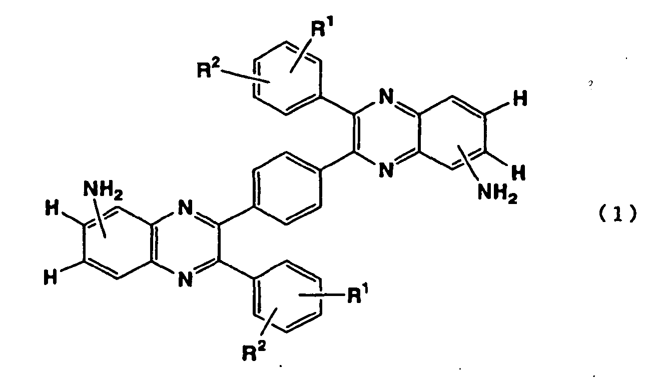 (where R¹ and R² each independently denotes a hydrogen atom, alkyl group, or alkoxyl group.)

2. The diaminobenzene compound as defined in claim 1, wherein R¹ and R² each independently denotes a C₁₋₂₀ alkyl group, C₁₋₂₀ alkoxyl group, or C₁₋₂₀ fluoroalkyl group.

3. A polyimide precursor which comprises repeating units represented by formula (2) below. 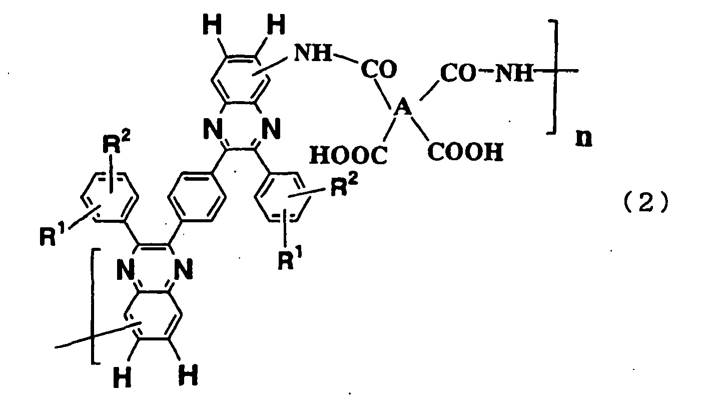 (where R¹ and R² each independently denotes a hydrogen atom, alkyl group, or alkoxyl group; "A" denotes a residue of tetracarboxylic acid; and n denotes an integer of 1 to 5000.)

4. A polyimide which comprises repeating units represented by formula (3) below. 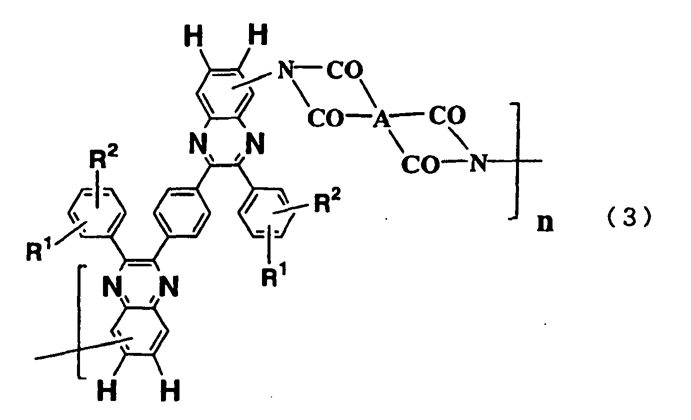 (where R¹ and R² each independently denotes a hydrogen atom, alkyl group, or alkoxyl group; "A" denotes a residue of tetracarboxylic acid; and n denotes an integer of 1 to 5000.)

5. A polyimide precursor which is obtained by reaction between a diamine component containing at least 1 mol% of the diaminobenzene compound defined in claim 1 or 2 and a tetracarboxylic acid or a derivative thereof.

6. The polyimide precursor as defined in claim 5, wherein the tetracarboxylic acid or the derivative thereof is an aromatic tetracarboxylic acid or a derivative thereof.

7. The polyimide precursor as defined in claim 6, wherein the aromatic tetracarboxylic acid is a tetracarboxylic acid having phenyl groups or substituted phenyl groups.

8. A polyimide which is obtained by ring-closing reaction from any of polyimide precursors as defined in claims 5 to 7.

9. A charge carrier transporting film which is formed from the polyimide as defined in claim 4 or 7.

10. An organic transistor device which is the charge carrier transporting film as defined in claim 9.

11. An organic light emitting diode which has at least one layer of the charge carrier transporting film as defined in claim 9.

12. A fluorescent filter which is the charge carrier transporting film as defined in claim 9.

13. A liquid crystal alignment film which is the charge carrier transporting film as defined in claim 9.

## Patentansprüche

1. Diaminobenzolverbindung der nachstehenden Formel (1): 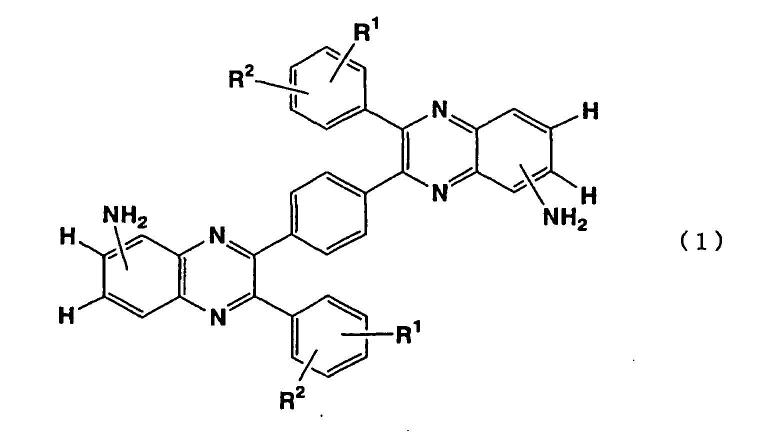 (worin R¹ und R² jeweils unabhängig voneinander für ein Wasserstoffatom, eine Alkylgruppe oder eine Alkoxylgruppe stehen).

2. Diaminobenzolverbindung nach Anspruch 1, worin R¹ und R² jeweils unabhängig voneinander für eine C₁₋₂₀-Alkylgruppe, eine C₁₋₂₀-Alkoxylgruppe oder eine C₁₋₂₀-Fluoralkylgruppe stehen.

3. Polyimidvorläufer, der Grundeinheiten der nachstehenden Formel (2) umfasst: 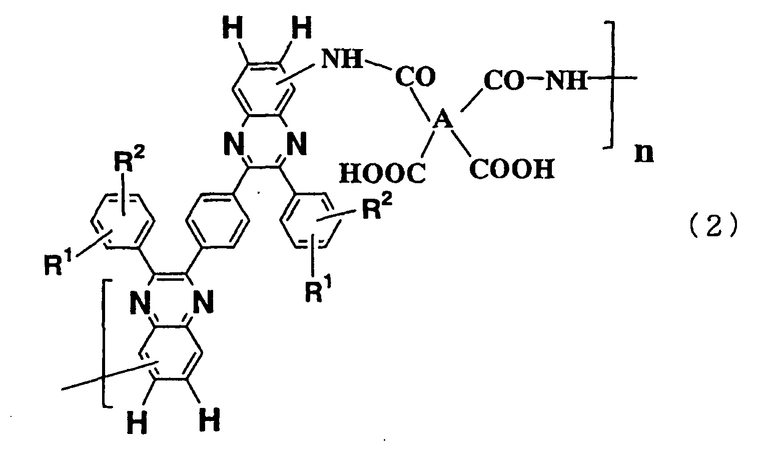 (worin R¹ und R² jeweils unabhängig voneinander für ein Wasserstoffatom, eine Alkylgruppe oder eine Alkoxylgruppe stehen; "A" für einen Rest einer Tetracarbonsäure steht; und n für eine ganze Zahl von 1 bis 5000 steht).

4. Polyimid, das Grundeinheiten der nachstehenden Formel (3) umfasst: 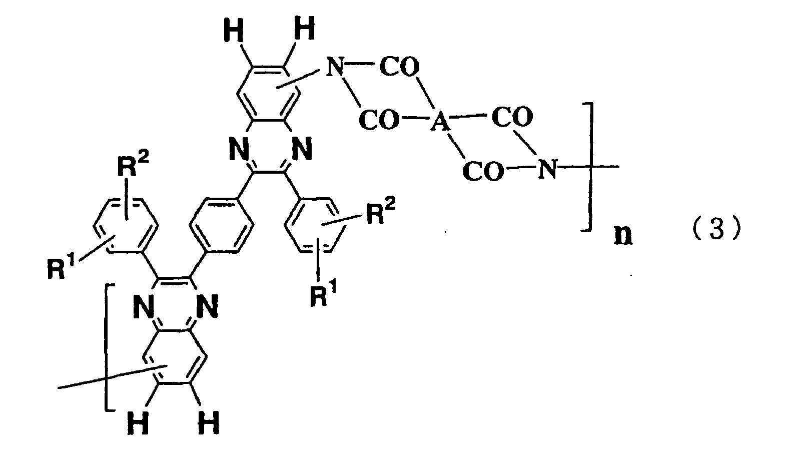 (worin R¹ und R² jeweils unabhängig voneinander für ein Wasserstoffatom, eine Alkylgruppe oder eine Alkoxylgruppe stehen; "A" für einen Rest einer Tetracarbonsäure steht; und n für eine ganze Zahl von 1 bis 5000 steht).

5. Polyimidvorläufer, der durch Umsetzung einer Diaminkomponente, die zumindest 1 Mol-% einer Diäminobenzolverbindung nach Anspruch 1 oder 2 enthält, mit einer Tetracarbonsäure oder einem Derivat davon erhältlich ist.

6. Polyimidvorläufer nach Anspruch 5, worin die Tetracarbonsäure oder das Derivat davon eine aromatische Tetracarbonsäure oder ein Derivat davon ist.

7. Polyimidvorläufer nach Anspruch 6, worin die aromatische Tetracarbonsäure eine Tetracarbonsäure ist, die Phenylgruppen oder substituierte Phenylgruppen aufweist.

8. Polyimid, das durch eine Ringschlussreaktion eines der in einem der Ansprüche 5 bis 7 definierten Polyimidvorläufer erhältlich ist.

9. Ladungsträgertransportfilm, der aus einem Polyimid nach einem der Ansprüche 4 oder 7 gebildet ist.

10. Organische Transistorvorrichtung, die ein Ladungsträgertransportfilm nach Anspruch 9 ist.

11. Organische Leuchtdiode, die zumindest eine Schicht aus einem Ladungsträgertransportfilm nach Anspruch 9 aufweist.

12. Fluoreszenzfilter, der ein Ladungsträgertransportfilm nach Anspruch 9 ist.

13. Flüssigkristallausrichtungsfilm, der ein Ladungsträgertransportfilm nach Anspruch 9 ist.

## Revendications

1. Composé de diaminobenzène représenté par la formule (1) ci-dessous: 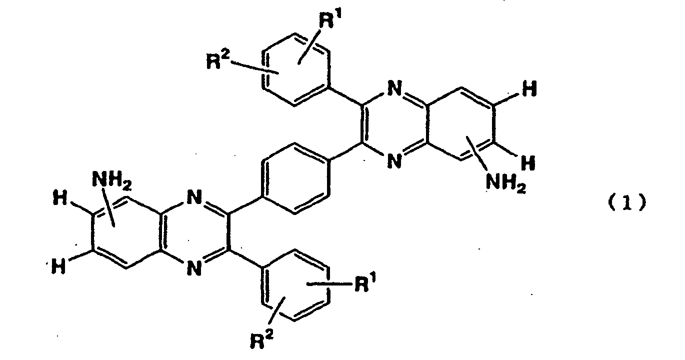 (où R¹ et R² indiquent chacun indépendamment un atome d'hydrogène, groupe alkyle ou groupe alcoxyle).

2. Composé de diaminobenzène tel que défini dans la revendication 1, où R¹ et R² indiquent chacun indépendamment un groupe alkyle C₁₋₂₀, groupe alkyle C₁₋₂₀ ou groupe fluoroalkyle C₁₋₂₀.

3. Précurseur de polyimide qui comprend des unités de répétition représentées par la formule (2) ci-dessous: 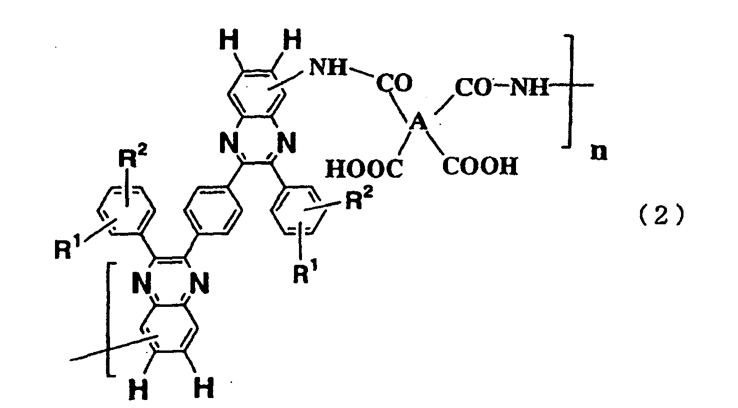 (où R¹ et R² indiquent chacun indépendamment un atome d'hydrogène, groupe alkyle ou groupe alcoxyle; "A" indique un résidu d'acide tétracarboxylique; et n indique un entier de 1 à 5000).

4. Polyimide qui comprend des unités de répétition représentées par la formule (3) ci-dessous 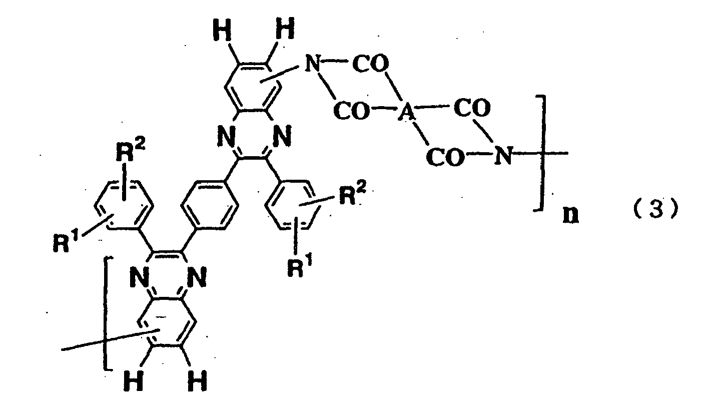 (où R¹ et R² indiquent chacun indépendamment un atome d'hydrogène, groupe alkyle ou groupe alcoxyle: "A" indique un résidu d'acide tétracarboxylique; et n indique un entier de 1 à 5000).

5. Précurseur de polyimide qui est obtenu par une réaction entre un composant de diamine contenant au moins 1% en mole du composé de diaminobenzène défini dans la revendication 1 ou 2 et un acide tétracarboxylique ou un dérivé de celui-ci.

6. Précurseur de polyimide tel que défini dans la revendication 5, où l'acide tétracarboxylique ou son dérivé est un acide tétracarboxylique aromatique ou un dérivé de celui-ci.

7. Précurseur de polyimide tel que défini dans la revendication 6, où l'acide tétracarboxylique aromatique est un acide tétracarboxylique ayant des groupes phényles ou des groupes phényles substitués.

8. Un polyimide qui est obtenu par une réaction de cyclisation à partir d'un quelconque des précurseurs de polyimide tels que définis dans les revendications 5 à 7.

9. Film de transport de support de charge qui est formé à partir du polyimide tel que défini dans la revendication 4 ou 7.

10. Dispositif de transistor organique qui est le film de transport de support de charge tel que défini dans la revendication 9.

11. Diode émettrice de lumière organique qui possède au moins une couche du film de transport de support de charge tel que défini dans la revendication 9.

12. Filtre fluororescent qui est le film de transport de support de charge tel que défini dans la revendication 9.

13. Film d'alignement de cristaux liquides qui est le film de transport de support de charge tel que défini dans la revendication 9.
